(19) Europäisches Patentamt
European Patent Office
Office européen des brevets

(11) Publication number: **0 189 254**
**A1**

(12) **EUROPEAN PATENT APPLICATION**

(21) Application number: 86300118.6

(22) Date of filing: 09.01.86

(51) Int. Cl.⁴: **C 07 D 401/12**, C 07 D 401/14, C 07 D 405/14, C 07 D 409/14, C 07 D 413/14, C 07 D 417/14, A 61 K 31/505

(30) Priority: 19.01.85 GB 8501395

(43) Date of publication of application: 30.07.86 Bulletin 86/31

(84) Designated Contracting States: AT BE CH DE FR GB IT LI LU NL SE

(71) Applicant: **Pfizer Limited, Ramsgate Road, Sandwich Kent CT13 9NJ (GB)**

(84) Designated Contracting States: GB

(71) Applicant: **Pfizer Corporation, Calle 15 1/2 Avenida Santa Isabel, Colon (PA)**

(84) Designated Contracting States: BE CH DE FR IT LI LU NL SE AT

(72) Inventor: **Alker, David, Dr., 1, Clive Road, Margate Kent (GB)**
Inventor: **Cross, Peter Edward, Dr., 21, Cherry Avenue, Canterbury Kent (GB)**

(74) Representative: **Moore, James William, Dr., Pfizer Limited Ramsgate Road, Sandwich Kent CT13 9NJ (GB)**

(54) **Dihydropyridine anti-ischaemic and antihypertensive agents.**

(57) 1,4-dihydropyridine derivatives of the formula (I):

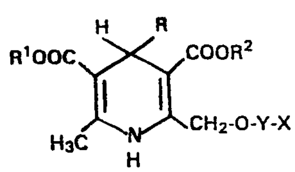

(I)

and pharmaceutically acceptable salts;
wherein
R is aryl or heteroaryl;
$R^1$ and $R^2$ are each independently $C_1$-$C_4$ alkyl or 2-methoxyethyl; Y is $-(CH_2)_n-$, $-CH_2CH(CH_3)-$ or $-CH_2C(CH_3)_2-$;
X is a group of the formula:

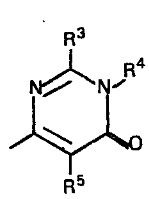

or

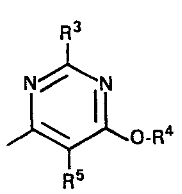

wherein
$R^3$ is H, het, $-(CH_2)_n$-het, -NH-het, $-NH-(CH_2)_n$-het, -O-het, $-O-(CH_2)_n$-het, $-(CH_2)_n-OH$, $-NR^6R^7$, $-(CH_2)_nNR^6R^7$ or $-NH-(CH_2)_pNR^6R^7$;
$R^4$ is H, $C_1$-$C_4$ alkyl, $-(CH_2)_pNR^6R^7$, $-(CH_2)_p-OH$ or $-(CH_2)_n$-het;
and
$R^5$ is H, $C_1$-$C_4$ alkyl or phenyl;
wherein
$R^6$ and $R^7$ are each independently H or $C_1$-$C_4$ alkyl or the two groups $R^6$ and $R^7$ are taken together with the nitrogen atom to which they are attached to form a pyrrolidinyl, piperidino, morpholino, piperazinyl or N-($C_1$-$C_4$ alkyl)piperazinyl group;
n is 1-4 and p is 2-4;
with the proviso that when $R^4$ is H or $C_1$-$C_4$ alkyl, $R^3$ is not H or $-NR^6R^7$.

These compounds are useful as anti-ischaemic and antihypertensive agents.

EP 0 189 254 A1

This invention relates to certain dihydropyridines, specifically to certain 1,4-dihydropyridines having a substituted pyrimidinyl group in a side chain attached to the 2-position which have utility as anti-ischaemic and antihypertensive agents, and to pharmaceutical preparations containing such compounds.

The compounds of the invention reduce the movement of calcium into the cell and they are thus able to delay or prevent the cardiac contracture which is believed to be caused by an accumulation of intracellular calcium under ischaemic conditions. Excessive calcium influx during ischaemia can have a number of additional adverse effects which would further compromise the ischaemic myocardium. These include less efficient use of oxygen for ATP production, activation of mitochondrial fatty acid oxidation and possibly, promotion of cell necrosis. Thus the compounds are useful in the treatment or prevention of a variety of cardiac conditions, such as angina pectoris, cardiac arrhythmias, heart attacks and cardiac hypertrophy. The compounds also have vasodilator activity since they can inhibit calcium influx in cells of vascular tissue and they are thus also useful as antihypertensive agents and for the treatment of coronary vasospasm.

According to the specification of our European patent application publication No. 60674 there are described and claimed a number of dihydropyridine anti-ischaemic and antihypertensive agents wherein the 2-position of the dihydropyridine ring is substituted with certain N,N-disubstituted aminoalkoxymethyl

groups. Our co-pending European patent application publication no. 100189 describes and claims a related series of compounds wherein the 2-position is substituted with an aromatic heterocyclylalkoxymethyl group. Our co-pending European patent application no. 84304888.5 discloses a further series of dihydropyridine compounds wherein the 2-position substituent bears a hydroxy- or oxo-substituted heterocyclic group. We have now discovered a further related series of dihydropyridine compounds having valuable therapeutic properties wherein the substituent in the 2-position bears a pyrimidine group having specific substituents.

Thus according to the invention, there are provided novel 1,4-dihydropyridine derivatives of the formula:-

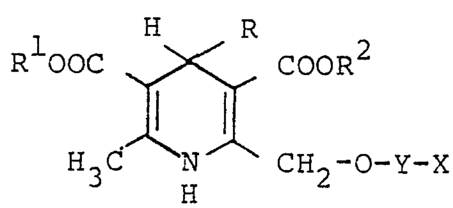

--- (I)

and pharmaceutically acceptable salts thereof;

wherein R is aryl or heteroaryl;

$R^1$ and $R^2$ are each independently $C_1$-$C_4$ alkyl or 2-methoxyethyl; Y is $-(CH_2)_n-$, $-CH_2CH(CH_3)-$ or $-CH_2C(CH_3)_2-$;

X is a group of the formula:

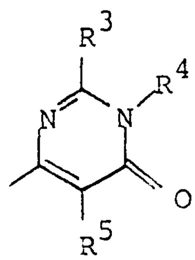

or

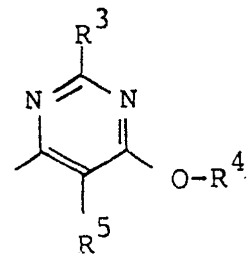

wherein $R^3$ is H, het, $-(CH_2)_n$-het, -NH-het, -NH-$(CH_2)_n$-het, -O-het, -O-$(CH_2)_n$-het, $-(CH_2)_n$-OH, $-NR^6R^7$, $-(CH_2)_nNR^6R^7$ or -NH-$(CH_2)_pNR^6R^7$;

$R^4$ is H, $C_1$-$C_4$ alkyl, $-(CH_2)_pNR^6R^7$, $-(CH_2)_p$-OH or $-(CH_2)_n$-het;

and $R^5$ is H, $C_1$-$C_4$ alkyl or phenyl;

wherein $R^6$ and $R^7$ are each independently H or $C_1$-$C_4$ alkyl or the two groups $R^6$ and $R^7$ are taken together with the nitrogen atom to which they are attached to form a pyrrolidinyl, piperidino, morpholino, piperazinyl or N-($C_1$-$C_4$ alkyl)piperazinyl group;

n is 1-4 and p is 2-4;

with the proviso that when $R^4$ is H or $C_1$-$C_4$ alkyl, $R^3$ is not H or $-NR^6R^7$.

The pharmaceutically acceptable acid addition salts of the compounds of the formula (I) are those formed from acids which form non-toxic acid addition salts, for example the hydrochloride, hydrobromide, sulphate or bisulphate, phosphate or acid phosphate,

acetate, citrate, fumarate, gluconate, lactate, maleate, succinate and tartrate salts. For compounds in which $R^4$ is H salts may also be formed with bases, examples are the sodium, potassium and ammonium salts.

The term "aryl" as used in this specification when R is phenyl or phenyl substituted by one or two substituents each independently selected from nitro, halo, $C_1$-$C_4$ alkyl, $C_1$-$C_4$ alkoxy, hydroxy, trifluoromethyl and cyano; or is 1- or 2-naphthyl.

The term "heteroaryl" as used in this specification when R is benzofuranyl; benzothienyl; pyridyl optionally substituted by methyl, methylthio, cyano or halo; quinolyl; benzoxazolyl; benzothiazolyl; furyl; pyrimidinyl; thiazolyl; 2,1,3-benzoxadiazol-4-yl; 2,1,3-benzothiadiazol-4-yl; or thienyl optionally monosubstituted by halo or $C_1$-$C_4$ alkyl.

As will be understood by those skilled in the art the group X can be subject to keto-enol tautomerism and the oxo group may be present either as a free hydroxyl group when in the tautomeric enol form, or as an oxo group when in the keto form. Which particular isomer is present in normal circumstances can be readily determined by appropriate physical measurements, such as for example by infra-red spectroscopy, and, in some instances the compounds may exist as mixtures of the two forms. However it is to be understood that the invention includes both forms of the compounds where they can exist and, in the description and Examples hereto, references to the hydroxy or keto form of the

compound include all possible tautomeric forms and are not necessarily to be taken to indicate which is the predominant tautomeric form of the compound.

The term "het" refers to a 5 or 6 membered nitrogen containing aromatic heterocyclic ring which may optionally be substituted with for example, $C_1$-$C_4$ alkyl or $C_1$-$C_4$ alkoxy groups. Examples of "het" groups include pyridyl or imidazolyl such as 2-pyridyl, 4-pyridyl or 1-methyl-2-imidazolyl.

Examples of preferred substituents for $R^3$ include: amino, aminomethyl, hydroxyethyl, 2-pyridyl, 4-morpholino-methyl, 2-pyridylmethylamino, or 4-pyridylmethylamino. $R^5$ is preferably hydrogen.

Examples of suitable substituents for $R_4$ include hydrogen, 2-(dimethylamino)ethyl, (1-methyl-2-imidazolyl)methyl, 2-hydroxyethyl or 2-pyridylmethyl.

The term "halo" means fluoro, chloro, bromo or iodo. Alkyl and alkoxy groups having 3 or more carbon atoms can be straight or branched chain. Compounds containing asymmetric centres will exist as one or more pairs of enantiomers and the invention includes the separated d- and l- optically active isomers as well as mixtures thereof.

Preferred values for R are 2-chlorophenyl and 2,3-dichlorophenyl. Preferably $R^1$ is $CH_3$ and $R^2$ is $C_2H_5$ or $R^1$ is $C_2H_5$ and $R^2$ is $CH_3$ and Y is $-(CH_2)-$. Thus particular and preferred examples of compounds of the invention include the following:

6-{[4-(2,3-Dichlorophenyl)-3-ethoxycarbonyl-5-methoxycarbonyl-6-methyl-1,4-dihydropyrid-2-yl]methoxymethyl}-2-(2-pyridylmethylamino)-4-pyrimidone.

2-Amino-6-{[4-(2,3-dichlorophenyl)-3-ethoxycarbonyl-5-methoxycarbonyl-6-methyl-1,4-dihydropyrid-2-yl]methoxymethyl}-3-(2-pyridylmethyl)-4-pyrimidone.

2-Amino-6-{[4-(2,3-dichlorophenyl)-3-ethoxycarbonyl-5-methoxycarbonyl-6-methyl-1,4-dihydropyrid-2-yl]methoxymethyl}-3[(1-methyl-2- imidazolyl)methyl]-4-pyrimidone.

2-Amino-6-{[4-(2,3-dichlorophenyl)-3-ethoxycarbonyl-5-methoxycarbonyl-6-methyl-1,4-dihydropyrid-2-yl]methoxymethyl}-4-(2-pyridylmethoxy)pyrimidine.

The compounds of formula (I) may be prepared by a number of different processes according to the invention.

(a) In one process, compounds of the formula (I) wherein $R^4$ is hydrogen may be prepared from the corresponding dihydropyridine ketoester of formula:

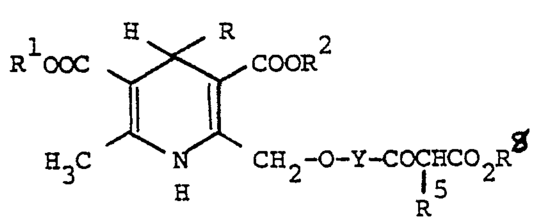

--- (II)

wherein R, $R^1$, $R^2$, $R^5$ and Y are as previously defined and $R^8$ is a $C_1$-$C_4$ alkyl group, by reacting with a compound of the formula

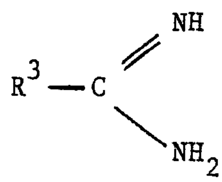

--- (III)

wherein $R^3$ is as previously defined other than H and $NH_2$.

The reaction of the compounds of formulae II and III is generally carried out by stirring the reactants, in more or less equimolar amounts, in an organic solvent in the presence of a base. Suitable solvents are lower alkanols such as ethanol. A period of several days at room temperature may be required before the reaction is substantially complete and the product is then isolated conventionally using normal extraction, washing and evaporation techniques and purified if necessary by recrystallisation or by chromatography.

The starting ketoesters of formula (II) are prepared using the procedures described in our European patent application no. 84304888.5 (publication no. 132375). The amidines of formula

(III) are generally known compounds, either commercially available or preparable by conventional methods in accordance with literature precedents.

(b) In an alternative process compounds of the formula (I) wherein $R^4$ is hydrogen and $R^3$ is $-NH(CH_2)_n$-het or $-NH(CH_2)_pNR^6R^7$ and n, p, het, $R^6$ and $R^7$ are as previously defined, are prepared from a compound of the formula:

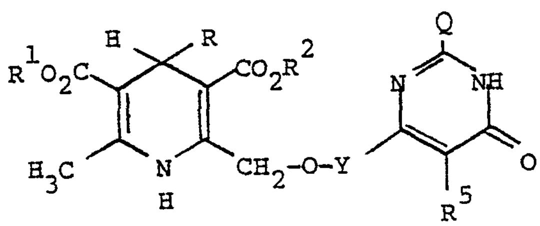

--- (IV)

wherein R, $R^1$, $R^2$, $R^5$ and Y are as previously defined and Q is $-NHNO_2$ or $-S(C_1-C_4$ alkyl) by reaction with an amine of the formula:

$$NH_2(CH_2)_n\text{het or } NH_2(CH_2)_pNR^6R^7$$

The displacement reaction is generally achieved by heating the compound of formula (IV) with excess amine at a temperature of 50-100°C. A period of up to 60 hours may be required for the

reaction to go substantially to completion and the product is then isolated and purified by conventional means as previously described.

The starting materials of formula (IV) are prepared by the process described under (a) using the ketoester of formula (II) and the compound of formula (III) wherein $R^3$ is $-NHNO_2$ or $-S(C_1-C_4$ alkyl), respectively.

(c) In a further process for the preparation of the compounds of formula (I) wherein $R^4$ is hydrogen and $R^3$ is $-(CH_2)_nNR^6R^7$, a compound of the formula (IV) wherein Q is $-(CH_2)_nCl$ is reacted with ammonia or an amine of the formula $HNR^6R^7$ wherein $R^6$ and $R^7$ are as previously defined other than hydrogen.

The reaction is particularly suitable for preparing the compounds wherein n is 1 and $R^6$ and $R^7$ are both hydrogen. Thus, in this case, the appropriate 2-chloromethylpyrimidone is simply reacted with concentrated ammonia solution at room temperature for several hours to yield the corresponding 2-aminomethyl derivative ($R^3 = CH_2NH_2$).

The starting materials of formula (IV) wherein Q is $-(CH_2)_nCl$ are again readily prepared from the ketoester of formula (II) by reaction with the appropriate ω-chloroalkanoamidine, by the method described under process (a).

(d) Compounds of the formula (I) wherein $R^4$ is other than hydrogen are prepared from a compound of the formula:

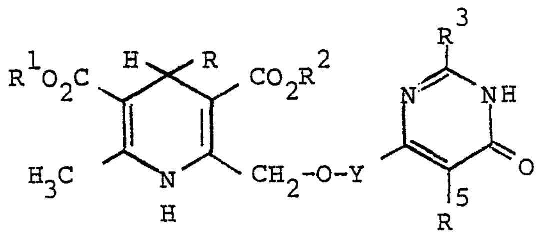

--- (V)

wherein R, $R^1$, $R^2$, $R^3$, $R^5$ and Y are as previously defined, by alkylation with a compound of the formula:

$R^4$-hal

wherein $R^4$ is as previously defined other than hydrogen and hal is a halogen atom, preferably chlorine.

Substitution takes place either on the N-3 ring nitrogen atom to give compounds of the formula (I) wherein X is:

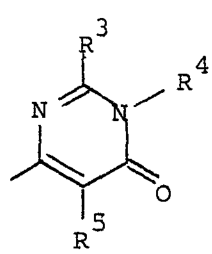

--- (a)

or on the 4-keto oxygen atom to give compounds of the formula (I) wherein X is:

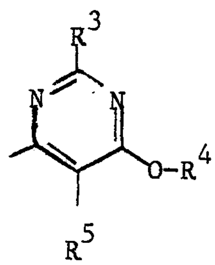

--- (b)

Appropriate conditions to obtain the N-substituted or O-substituted product are well known to those skilled in the art. Thus alkylation with the appropriate halo compound in a protic solvent, e.g. N,N-dimethylformamide in the presence of a weak base yields the N-substituted product wherein X is (a). Alternatively, alkylation in an aprotic solvent, e.g. toluene, in the presence of silver carbonate yields the O-substituted product wherein X is (b). In either case the products are isolated and purified using conventional procedures as previously defined.

In some cases mixtures of two products may be obtained. These may be separated by conventional means e.g. crystallisation or chromatography.

The ability of the compounds to inhibit the movement of calcium into the cell is shown by their effectiveness in reducing the response of isolated heart tissue to an increase in calcium ion concentration in vitro. The test is performed by mounting spirally cut strips of rat aorta with one end fixed and the other attached to a force transducer. The tissue is immersed in a bath of physiological saline solution containing potassium ions at a concentration of 45 millimolar and no calcium. Calcium chloride

is added to the bath with a pipette to give a final calcium ion concentration of 2 millimolar. The change in tension caused by the resulting contraction of the tissue is noted. The bath is drained and replaced with fresh saline solution and after 45 minutes the procedure is repeated with the particular compound under test present in the saline solution. The concentration of compound required to reduce the response by 50% is recorded.

The antihypertensive activity of the compounds is also evaluated after oral administration by measuring the fall in blood pressure in spontaneously hypertensive rats or renally hypertensive dogs.

For administration to man in the curative or prophylactic treatment of cardiac conditions and hypertension, oral dosages of the compounds will generally be in the range of from 2-100 mg daily for an average adult patient (70 kg). Thus for a typical adult patient, individual tablets or capsules contain from 1 to 10 mg of active compound, in a suitable pharmaceutically acceptable vehicle or carrier. Dosages for intravenous administration would typically be within the range 1 to 10 mg per single dose as required. In practice the physician will determine the actual dosage which will be most suitable for an individual patient and it will vary with the age, weight and response of the particular patient. The above dosages are exemplary of the average case but there can, of course, be individual instances where higher or lower dosage ranges are merited, and such are within the scope of this invention.

For human use, the compounds of the formula (I) can be administered alone, but will generally be administered in admixture with a pharmaceutical carrier selected with regard to the intended route of administration and standard pharmaceutical practice. For example, they may be administered orally in the form of tablets containing such excipients as starch or lactose, or in capsules or ovules either alone or in admixture with excipients, or in the form of elixirs or suspensions containing flavouring or colouring agents. They may be injected parenterally, for example, intravenously, intramuscularly or subcutaneously. For parenteral administration, they are best used in the form of a sterile aqueous solution which may contain other substances, for example, enough salts or glucose to make the solution isotonic with blood.

Thus in a further aspect the invention provides a pharmaceutical composition comprising a compound of the formula (I), or a pharmaceutically acceptable salt thereof, together with a pharmaceutically acceptable diluent or carrier.

The invention also provides a compound of the formula (I) or a pharmaceutically acceptable salt thereof, for use in the treatment of cardiovascular conditions including use in the prevention or treatment of cardiac conditions, or use as an antihypertensive, in man.

The preparation of the compounds of the invention is illustrated by the following Examples.

## EXAMPLE 1

**6-{[4-(2,3-Dichlorophenyl)-3-ethoxycarbonyl-5-methoxycarbonyl-6-methyl-1,4-dihydropyrid-2-yl]methoxymethyl}-2-hydroxymethyl-4-pyrimidone**

A solution of ethyl 4-{[4-(2,3-dichlorophenyl)-3-ethoxycarbonyl-5-methoxycarbonyl-6-methyl-1,4-dihydropyrid-2-yl]methoxy}-acetoacetate (0.53 g), 2-hydroxyacetamidine hydrochloride (0.13 g) and 1,5-diazabicyclo[4.3.0]non-5-ene (0.13 g) in ethanol (20 ml) was stirred at room temperature for three days and evaporated. The residue was dissolved in chloroform and the solution washed with 0.1 M hydrochloric acid, water and saturated aqueous sodium hydrogen carbonate solution, dried ($Na_2SO_4$) and evaporated. The residue was crystallised from diethyl ether/ethyl acetate to give the title compound (0.24 g), m.p. 190-193°C.

Analysis %:-

| | |
|---|---|
| Found: | C,53.27; H,4.76; N,7.88; |
| $C_{24}H_{25}Cl_2N_3O_7$ requires: | C,53.53; H,4.68; N,7.80%. |

## EXAMPLE 2

6-{[4-(2,3-Dichlorophenyl)-3-ethoxycarbonyl-5-methoxycarbonyl-6-methyl-1,4-dihydropyrid-2-yl]methoxymethyl}-2-(2-pyridyl)-4-pyrimidone was prepared by the method described in Example 1 using pyridine-2-carboxamidine as the starting material. The product had m.p. 169-170°C.

Analysis %:-

| | |
|---|---|
| Found: | C,57.23; H,4.69; N,9.54; |
| $C_{28}H_{26}Cl_2N_4O_6$ requires: | C,57.44; H,4.48; N,9.57%. |

EXAMPLE 3

6-{[4-(2,3-Dichlorophenyl)-5-ethoxycarbonyl-3-methoxycarbonyl-6-methyl-1,4-dihydropyrid-2-yl]methoxymethyl}-2-(2-pyridyl)-4-pyrimidone was prepared by the method described in Example 2 using ethyl 4-{[4-(2,3-dichlorophenyl)-5-ethoxycarbonyl-3-methoxycarbonyl-6-methyl-1,4-dihydropyrid-2-yl]methoxy}-acetoacetate as the starting material. The product had m.p. 192-193°C.

Analysis %:-

| | |
|---|---|
| Found: | C,56.98; H,4.61; N,9.09; |
| $C_{28}H_{26}Cl_2N_4O_6$ requires: | C,57.44; H,4.48; N,9.57%. |

EXAMPLE 4

6-{[4-(2,3-Dichlorophenyl)-3-ethoxycarbonyl-5-methoxycarbonyl-6-methyl-1,4-dihydropyrid-2-yl]methoxymethyl}-2-(4-morpholinomethyl)-4-pyrimidone was prepared by the method described in Example 1 using 2-(4-morpholino)acetamidine as the starting material. The product had m.p. 130-135°C.

Analysis %:-

| | |
|---|---|
| Found: | C,55.59; H,5.65; N,8.77; |
| $C_{28}H_{32}Cl_2N_4O_7$ requires: | C,55.36; H,5.31; N,9.22%. |

EXAMPLE 5

6-{[4-(2,3-Dichlorophenyl)-3-ethoxycarbonyl-5-methoxycarbonyl-6-methyl-1,4-dihydropyrid-2-yl]methoxymethyl}-2-(2-pyridylmethylamino)-4-pyrimidone

A mixture of 2-aminomethylpyridine (2.0 g) and 6-{[4-(2,3-dichlorophenyl)-3-ethoxycarbonyl-5-methoxycarbonyl-6-methyl-1,4-dihydropyrid-2-yl]methoxymethyl}-2-nitramino-4-pyrimidone (0.50 g) was heated at 75°C for 7 hours, dissolved in ethyl acetate (100 ml), washed three times with water, dried ($MgSO_4$) and evaporated. The residue was purified by chromatography on silica (5 g) using dichloromethane plus 0-1% v/v methanol as eluant. Appropriate fractions were combined and evaporated and the residue crystallised from diethyl ether and recrystallised from ethyl acetate to give the title compound (0.17 g), m.p. 175-177°C.

Analysis %:-

| | |
|---|---|
| Found: | C,56.75; H,4.84; N,11.42; |
| $C_{29}H_{29}Cl_2N_5O_6$ requires: | C,56.68; H,4.76; N,11.40%. |

EXAMPLE 6

6-{[4-(2,3-Dichlorophenyl)-3-ethoxycarbonyl-5-methoxycarbonyl- 6-methyl-1,4-dihydropyrid-2-yl]methoxymethyl}-2-(4-pyridylmethylamino)-4-pyrimidone

A mixture of 6-{[4-(2,3-dichlorophenyl)-3-ethoxycarbonyl-5-methoxycarbonyl-6-methyl-1,4-dihydropyrid-2-yl]methoxymethyl}-2-methylthio-4-pyrimidone (0.50 g) and 4-aminomethylpyridine (5.0 ml) was heated at 95°C for 60 hours and then dissolved in

chloroform. The resulting solution was washed twice with dilute hydrochloric acid at pH5, dried ($MgSO_4$) and evaporated. The residue was purified twice by chromatography on silica (6 g) using dichloromethane plus 0-4% v/v methanol as eluant followed by chloroform plus 1% acetic acid as eluant. Appropriate fractions were combined and evaporated and the residue triturated with diethyl ether to give the title compound (80 mg) as a hemihydrate, m.p. 140-147°C.

Analysis %:-

| | |
|---|---|
| Found: | C,55.68; H,4.77; N,11.11; |
| $C_{29}H_{29}Cl_2N_5O_6$.0.5 $H_2O$ requires: | C,55.86; H,4.85; N,11.23%. |

EXAMPLE 7

2-Amino-6-{[4-(2,3-dichlorophenyl)-3-ethoxycarbonyl-5-methoxycarbonyl-6-methyl-1,4-dihydropyrid-2-yl]methoxymethyl}-3-(2-pyridylmethyl)-4-pyrimidone

A solution of 2-amino-6-{[4-(2,3-dichlorophenyl)-3-ethoxycarbonyl-5-methoxycarbonyl-6-methyl-1,4-dihydropyrid-2-yl]methoxymethyl}-4-pyrimidone (0.53 g) and 2-chloromethylpyridine (0.52 g) in N,N-dimethylformamide (20 ml) containing potassium carbonate (0.45 g) was stirred at room temperature for 8 days and then evaporated. The residue was partitioned between chloroform and water and the organic layer washed twice with water, dried ($MgSO_4$) and evaporated. The residue was crystallised from diethyl ether to give the title compound (102 mg), m.p. 122-125°C.

Analysis %:-

Found: C,56.78; H,4.87; N,11.15;

$C_{29}H_{29}Cl_2N_5O_6$ requires: C,56.68; H,4.76; N,11.40%.

EXAMPLES 8-10

The following compounds were prepared by the method described in Example 7 by reacting 2-amino-6 {[4-(2,3-dichlorophenyl)-3-ethoxycarbonyl-5-methoxycarbonyl-6-methyl-1,4-dihydropyrid-2-yl]methoxymethyl}-4-pyrimidone in N,N-dimethylformamide solution with the appropriate alkylating agent in the presence of potassium carbonate. The products were characterised in the form indicated.

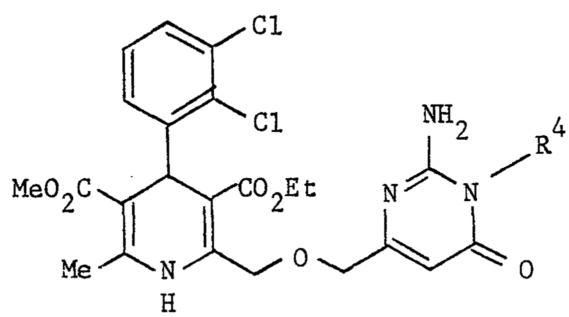

| Example No. | $R^4$ | m.p. (°C) | Form characterised | Analysis % (theoretical in brackets) C | H | N |
|---|---|---|---|---|---|---|
| 8 | $.CH_2CH_2NMe_2$ | 135-138 | hydrate | 53.53<br>(52.94 | 5.56<br>5.76 | 11.46<br>11.43) |
| 9 | 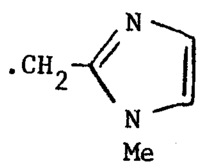 | 130-145 | + one mole of toluene of crystallisation | 58.98<br>(59.23 | 5.55<br>5.40 | 11.88<br>11.84) |
| 10 | $.CH_2CH_2OH$ | 125-130 | free base | 52.33<br>(52.92 | 4.87<br>4.97 | 9.75<br>9.87) |

EXAMPLE 11

2-Amino-6-{[4-(2,3-dichlorophenyl)-3-ethoxycarbonyl-5-methoxycarbonyl-6-methyl-1,4-dihydropyrid-2-yl]methoxymethyl}-4-(2-pyridylmethoxy)pyrimidine

A mixture of 2-amino-6-{[4-(2,3-dichlorophenyl)-3-ethoxycarbonyl-5-methoxycarbonyl-6-methyl-1,4-dihydropyrid-2-yl]methoxymethyl}-4-pyrimidone (0.53 g), 2-chloromethylpyridine hydrochloride (0.34 g) and silver carbonate (1.10 g) in toluene (40 ml) was heated under reflux for three days and then evaporated. The residue was taken up in chloroform (100 ml) and filtered. The filtrate was washed three times with water, dried ($MgSO_4$) and evaporated. The residue was purified by chromatography on silica (6 g) using dichloromethane plus 0-4% v/v methanol as eluant. Appropriate fractions were combined and evaporated. The residue was crystallised from diethyl ether to give the title compound (80 mg), m.p. 152-154°C.

Analysis %:-

| | |
|---|---|
| Found: | C,56.40; H,4.81; N,11.17; |
| $C_{29}H_{29}Cl_2N_5O_6$ requires: | C,56.68; H,4.75; N,11.40%. |

EXAMPLE 12

2-Aminomethyl-6-{[4-(2,3-dichlorophenyl)-3-ethoxycarbonyl-5-methoxycarbonyl-6-methyl-1,4-dihydropyrid-2-yl]methoxymethyl}-4-pyrimidone

A mixture of 2-chloromethyl-6-{[4-(2,3-dichlorophenyl)-3-ethoxycarbonyl-5-methoxycarbonyl-6-methyl-1,4-dihydropyrid-2-yl]methoxymethyl}-4-pyrimidone (0.40 g) and .880 aqueous ammonia solution (40 ml) was stirred at room temperature for 16 hours and then evaporated. The residue was partitioned between chloroform and water and the organic layer dried ($MgSO_4$) and evaporated. The residue was crystallised from diethyl ether to give the title compound (60 mg), m.p. 105-110°C.

Analysis %:-

| | |
|---|---|
| Found: | C,52.98; H,4.75; N,10.21; |
| $C_{24}H_{26}Cl_2N_4O_6$ requires: | C,53.64; H,4.88; N,10.43%. |

## Preparation 1

Ethyl 4-{[4-(2,3-dichlorophenyl)-3-ethoxycarbonyl-5-methoxycarbonyl-6-methyl-1,4-dihydropyrid-2-yl]methoxy}acetoacetate

A solution of carbonyl diimidazole (5.20 g) and 2-{[4-(2,3-dichlorophenyl)-3-ethoxycarbonyl-5-methoxycarbonyl-6-methyl-1,4-dihydropyrid-2-yl]methoxy} acetic acid (14.00 g) in dichloromethane (200 ml) was stirred at room temperature under nitrogen for 2 hours and then added to a solution of pyridine (2.40 g) and 2,2-dimethyl-1,3-dioxane-4,6-dione (4.56 g) in dichloromethane (200 ml) over 8 minutes. The mixture was stirred at room temperature for 2.5 hours, washed successively with water, ice-cold 2.5 M hydrochloric acid and saturated brine, dried ($MgSO_4$) and evaporated. The residue was dissolved in ethanol (200 ml) and the solution heated under reflux for 2.75 hours and evaporated. The residue was triturated with diethyl ether and the

resulting solid collected, washed with diethyl ether and dried to give the title compound (9.0 g), m.p. 99-102°C.

## Preparation 2

Ethyl 4-{[4-(2,3-dichlorophenyl)-3-ethoxycarbonyl-5-methoxycarbonyl-6-methyl-1,4-dihydropyrid-2-yl]methoxy}acetoacetate was prepared by the method described in Preparation 1 using 2-{[4-(2,3-dichlorophenyl)-3-ethoxycarbonyl-5-methoxycarbonyl-6-methyl-1,4-dihydropyrid-2-yl]methoxy}acetic acid as the starting material. The compound obtained had a m.p. of 134-7°C.

Analysis %:

| | |
|---|---|
| Found: | C,54.44; H,5.21; N,2.64; |
| $C_{24}H_{27}Cl_2NO_8$ requires: | C,54.55; H,5.15; N,2.65%. |

## Preparation 3

2-Chloromethyl-6-{[4-(2,3-dichlorophenyl)-3-ethoxycarbonyl-5-methoxycarbonyl-6-methyl-1,4-dihydropyrid-2-yl]methoxymethyl}-4-pyrimidone

A solution of ethyl 4-{[4-(2,3-dichlorophenyl)-3-ethoxycarbonyl-5-methoxycarbonyl-6-methyl-1,4-dihydropyrid-2-yl]methoxy}acetoacetate (3.44 g), 2-chloroacetamidine hydrochloride (1.00 g) and 1,5-diazabicyclo[4.3.0]non-5-ene (1.3 g) in ethanol (40 ml) was stirred at room temperature for four days and then evaporated. The residue was partitioned between ethyl acetate and water and the organic layer washed with dilute hydrochloric acid at pH4, dried ($MgSO_4$) and evaporated. The

residue was purified by chromatography on silica (10 g) using dichloromethane plus 30% v/v hexane followed by dichloromethane plus 0-1% v/v methanol as eluant. Appropriate fractions were combined and evaporated and the residue crystallised from ethyl acetate to give the title compound (0.46 g), m.p. 198-200°C.

Analysis %:-

| | |
|---|---|
| Found: | C,52.27; H,4.35; N,7.69; |
| $C_{24}H_{24}Cl_3N_3O_6$ requires: | C,51.76; H,4.34; N,7.55%. |

## Preparation 4

6-{[4-(2,3-Dichlorophenyl)-3-ethoxycarbonyl-5-methoxycarbonyl-6-methyl-1,4-dihydropyrid-2-yl]methoxymethyl}-2-nitramino-4-pyrimidone was prepared by the method described in Preparation 1 using nitroguanidine as the starting material instead of 2-chloroacetamidine. The product was charaterised as its hydrate, m.p. 197-200°C decomp.

Analysis %:-

| | |
|---|---|
| Found: | C,47.42; H,4.09; N,11.79; |
| $C_{23}H_{23}Cl_2N_5O_8.H_2O$ requires: | C,47.11; H,3.95; N,11.95%. |

## Preparation 5

6-{[4-(2,3-Dichlorophenyl)-3-ethoxycarbonyl-5-methoxycarbonyl-6-methyl-1,4-dihydropyrid-2-yl]methoxymethyl}-2-methylthio-4-pyrimidone

A solution of ethyl 4-{[4-(2,3-dichlorophenyl)-3-ethoxycarbonyl-5-methoxycarbonyl-6-methyl-1,4-dihydropyrid-2-yl]methoxy} acetoacetate (1.00 g), S-methylisothiouronium sulphate (0.53 g) and 1,5-diazabicyclo[4.3.0]non-5-ene (0.35 g) in ethanol (30 ml) was stirred at room temperature for 5 days and then evaporated. The residue was partitioned between ethyl acetate and water and the organic layer washed with dilute hydrochloric acid at pH2, dried ($Na_2SO_4$) and evaporated. The residue was crystallised from diethyl ether to give the title compound (0.50 g), m.p. 230-234°C decomp.

Analysis %:-

| | |
|---|---|
| Found: | C,51.88; H,4.77; N,7.24; |
| $C_{24}H_{25}Cl_2N_3O_6S$ requires: | C,51.99; H,4.55; N,7.58%. |

Preparation 6

2-Amino-6-{[4-(2,3-dichlorophenyl)-3-ethoxycarbonyl-5-methoxycarbonyl-6-methyl-1,4-dihydropyrid-2-yl]methoxymethyl}-4-pyrimidone

A solution of ethyl 4-{[4-(2,3-dichlorophenyl)-3-ethoxycarbonyl-5-methoxycarbonyl-6-methyl-1,4-dihydropyrid-2-yl]methoxy}acetoacetate (0.74 g), 1,5-diazabicyclo[4.3.0]non-5-ene (0.25 g) and guanidine hydrochloride (0.14 g) in ethanol (30 ml) was heated under reflux for 5.5 hours and then evaporated. The residue was dissolved in chloroform and the solution washed successively with water, 10% aqueous sodium carbonate solution and water, dried

($MgSO_4$) and evaporated. The residue was triturated with ethyl acetate and the resulting precipitate collected, washed thoroughly with diethyl ether, recrystallised from ethyl acetate/ethanol and dried to give the title compound (0.20 g), m.p. 222-225°C.

Analysis %:-

| | |
|---|---|
| Found: | C,52.10; H,4.56; N,10.54. |
| $C_{23}H_{24}Cl_2N_4O_6$ requires: | C,52.78; H,4.62; N,10.70%. |

0189254



CLAIMS FOR THE CONTRACTING STATES:
BE, CH, DE, FR, GB, IT, LI, LU, NL, SE

1. 1,4-dihydropyridine derivatives of the formula (I):-

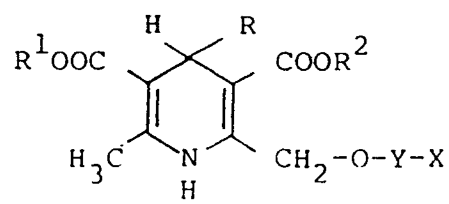

--- (I)

and pharmaceutically acceptable salts thereof;

wherein R is aryl or heteroaryl;

$R^1$ and $R^2$ are each independently $C_1$-$C_4$ alkyl or 2-methoxyethyl; Y is $-(CH_2)_n-$, $-CH_2CH(CH_3)-$ or $-CH_2C(CH_3)_2-$;

X is a group of the formula:

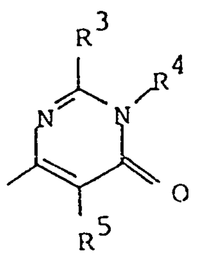

or

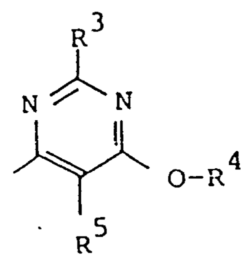

wherein $R^3$ is H, het, $-(CH_2)_n$-het, -NH-het, -NH-$(CH_2)_n$-het, -O-het, -O-$(CH_2)_n$-het, $-(CH_2)_n-OH$, $-NR^6R^7$, $-(CH_2)_nNR^6R^7$ or $-NH-(CH_2)_pNR^6R^7$;

$R^4$ is H, $C_1$-$C_4$ alkyl, $-(CH_2)_pNR^6R^7$, $-(CH_2)_p$-OH or $-(CH_2)_n$-het;

and $R^5$ is H, $C_1$-$C_4$ alkyl or phenyl;

wherein $R^6$ and $R^7$ are each independently H or $C_1$-$C_4$ alkyl or the two groups $R^6$ and $R^7$ are taken together with the nitrogen atom to which they are attached to form a pyrrolidinyl, piperidino, morpholino, piperazinyl or N-($C_1$-$C_4$ alkyl)piperazinyl group;

n is 1-4 and p is 2-4;

with the proviso that when $R^4$ is H or $C_1$-$C_4$ alkyl, $R^3$ is not H or $-NR^6R^7$.

2. A compound of formula (I) as claimed in claim 1 wherein R is aryl and aryl is phenyl or phenyl substituted by one or two substituents each independently selected from nitro, halo, $C_1$-$C_4$ alkyl, $C_1$-$C_4$ alkoxy, hydroxy, trifluoromethyl and cyano; or is 1- or 2-naphthyl; or wherein R is heteroaryl and heteroaryl is benzofuranyl; benzothienyl; pyridyl optionally substituted by methyl, methylthio, cyano or halo; quinolyl; benzoxazolyl; benzothiazolyl; furyl; pyrimidinyl; thiazolyl; 2,1,3-benzoxadiazol-4-yl; 2,1,3-benzothiadiazol-4-yl; or thienyl optionally monosubstituted by halo or $C_1$-$C_4$ alkyl.

3. A compound as claimed in claim 3 wherein R is 2-chlorophenyl or 2,3-dichlorophenyl.

4. A compound as claimed in any preceding claim wherein either $R^1$ is $CH_3$ and $R^2$ is $C_2H_5$ or $R^1$ is $C_2H_5$ and $R^2$ is $CH_3$ and Y is $-(CH_2)-$.

5. A compound as claimed in any preceding claim wherein het is a 5 or 6 membered nitrogen containing aromatic heterocyclic ring which may optionally be substituted with $C_1$-$C_4$ alkyl or $C_1$-$C_4$ alkoxy groups.

6. A compound as claimed in claim 5 wherein het is 2-pyridyl, 4-pyridyl or 1-methyl-2-imidazolyl.

7. A compound as claimed in any preceding claim wherein $R^3$ is amino, aminomethyl, hydroxyethyl, 2-pyridyl, 4-morpholinomethyl, 2-pyridylmethylamino, or 4-pyridylmethylamino.

8. A compound as claimed in any preceding claim wherein $R^4$ is hydrogen, 2-(dimethylamino)ethyl, (1-methyl-2-imidazolyl)-methyl, 2-hydroxyethyl or 2-pyridylmethyl.

9. A compound as claimed in any preceding claim wherein $R^5$ is hydrogen.

10. A pharmaceutical composition comprising a compound of formula (I) as claimed in any preceding claim, or a pharmaceutically acceptable acid addition salt thereof, together with a pharmaceutically acceptable diluent or carrier.

11. A compound of the formula (I) as defined in any one of claims 1 to 9, or a pharmaceutically acceptable addition salt thereof, for use in medicine.

CLAIMS FOR THE CONTRACTING STATE: AT

1. A process for preparing a compound of formula (I):-

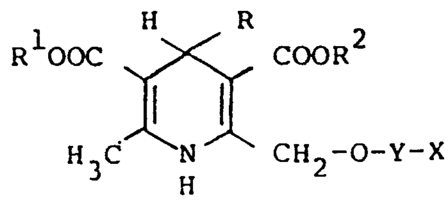 --- (I)

and pharmaceutically acceptable salts;

wherein R is aryl or heteroaryl;

$R^1$ and $R^2$ are each independently $C_1$-$C_4$ alkyl or 2-methoxyethyl; Y is $-(CH_2)_n-$, $-CH_2CH(CH_3)-$ or $-CH_2C(CH_3)_2-$;

X is a group of the formula:

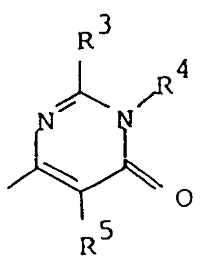

or

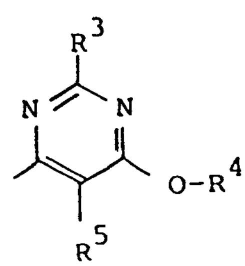

wherein $R^3$ is H, het, $-(CH_2)_n$-het, -NH-het, $-NH-(CH_2)_n$-het, -O-het, $-O-(CH_2)_n$-het, $-(CH_2)_n-OH$, $-NR^6R^7$, $-(CH_2)_nNR^6R^7$ or $-NH-(CH_2)_pNR^6R^7$;

$R^4$ is H, $C_1$-$C_4$ alkyl, $-(CH_2)_pNR^6R^7$, $-(CH_2)_p-OH$ or $-(CH_2)_n$-het;

and $R^5$ is H, $C_1$-$C_4$ alkyl or phenyl;

wherein $R^6$ and $R^7$ are each independently H or $C_1$-$C_4$ alkyl or the two groups $R^6$ and $R^7$ are taken together with the nitrogen atom to which they are attached to form a pyrrolidinyl, piperidino, morpholino, piperazinyl or N-($C_1$-$C_4$ alkyl)piperazinyl group;

n is 1-4 and p is 2-4;

with the proviso that when $R^4$ is H or $C_1$-$C_4$ alkyl, $R^3$ is not H or $-NR^6R^7$

characterised by:

a) reaction of a compound of formula (II)

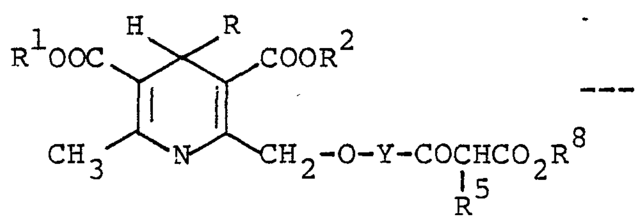

--- (II)

wherein R, $R^1$, $R^2$, $R^5$ and Y are as previously defined and $R^8$ is a $C_1$-$C_4$ alkyl group with a compound of the formula (III)

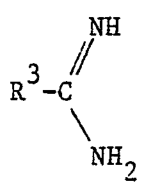

wherein $R^3$ is as previously defined other than H and $NH_2$.

or b) to give compounds of the formula (I) wherein $R^4$ is hydrogen and $R^3$ is $-NH(CH_2)_n$-het or $-NH(CH_2)_pNR^6R^7$ and n, p, het, $R^6$ and $R^7$ are as previously defined by reaction of a compound of formula (IV)

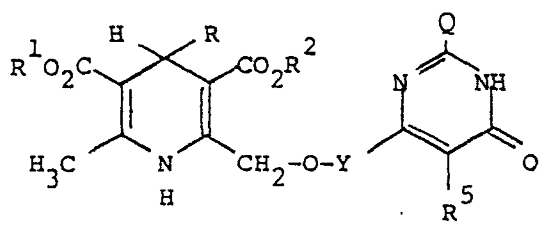 --- (IV)

wherein R, $R^1$, $R^2$, $R^5$ and Y are as previously defined and Q is $-NHNO_2$ or $-S(C_1-C_4$ alkyl) with an amine of the formula:

$$NH_2(CH_2)_n\text{het or } NH_2(CH_2)_pNR^6R^7$$

or c) to give compounds of formula (I) wherein $R^4$ is hydrogen and $R^3$ is $-(CH_2)_nNR^6R^7$, by reaction of a compound of the formula (IV) wherein Q is $-(CH_2)_nCl$ with ammonia or an amine of the formula $HNR^6R^7$ wherein $R^6$ and $R^7$ are as previously defined other than hydrogen.

or d) to give compounds of the formula (I) wherein $R^4$ is other than hydrogen by reaction of a compound of the formula (V):

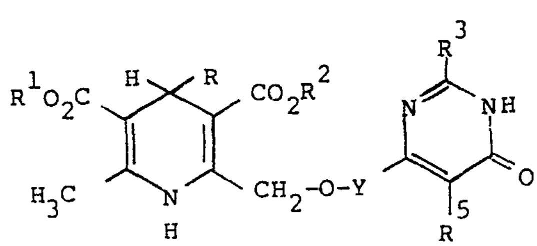 --- (V)

wherein R, $R^1$, $R^2$, $R^3$, $R^5$ and Y are as previously defined, with a compound of the formula:

$$R^4\text{-hal}$$

wherein $R^4$ is as previously defined other than hydrogen and hal is a halogen atom.

2. A process as claimed in claim 1 wherein R is 2-chlorophenyl or 2,3-dichlorophenyl.

3. A process as claimed in any preceding claim wherein either $R^1$ is $CH_3$ and $R^2$ is $C_2H_5$ or $R^1$ is $C_2H_5$ and $R^2$ is $CH_3$ and Y is $-(CH_2)-$.

4. A process as claimed in any preceding claim wherein het is a 5 or 6 membered nitrogen containing aromatic heterocyclic ring which may optionally be substituted with $C_1$-$C_4$ alkyl or $C_1$-$C_4$ alkoxy groups.

5. A process as claimed in claim 4 wherein het is 2-pyridyl, 4-pyridyl or 1-methyl-2-imidazolyl.

6. A process as claimed in any preceding claim wherein $R^3$ is aminomethyl, hydroxyethyl, 2-pyridyl, 4-morpholino-methyl, 2-pyridylmethylamino, or 4-pyridylmethylamino.

7. A process as claimed in any preceding claim wherein $R^4$ is hydrogen, 2-(dimethylamino)ethyl, (1-methyl-2-imidazolyl)methyl, 2-hydroxyethyl or 2-pyridylmethyl.

8. A process as claimed in any preceding claim wherein $R^5$ is hydrogen.

9. A process for preparing a pharmaceutical composition comprising mixing a compound of formula (I) as claimed in any preceding claim or a pharmaceutically acceptable salt thereof, with a pharmaceutically acceptable diluent or carrier.

0189254

European Patent Office

# EUROPEAN SEARCH REPORT

Application number

EP 86300118.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X, P | EP - A2 - 0 132 375 (PFIZER) * Claims 1,9 * -- | 1,10, 11 | C 07 D 401/12<br>C 07 D 401/14<br>C 07 D 405/14<br>C 07 D 409/14<br>C 07 D 413/14<br>C 07 D 417/14<br>A 61 K 31/505 |
| A | EP - A2 - 0 119 050 (PFIZER) * Claims 1,8; examples 22,29 * ---- | 1,10, 11 | |

| TECHNICAL FIELDS SEARCHED (Int. Cl.4) |
|---|
| C 07 D 401/00 |
| C 07 D 405/00 |
| C 07 D 409/00 |
| C 07 D 413/00 |
| C 07 D 417/00 |

The present search report has been drawn up for all claims

| Place of search | Date of completion of the search | Examiner |
|---|---|---|
| VIENNA | 04-04-1986 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82